# EUROPEAN PATENT APPLICATION

(11) **EP 4 635 528 A1**
(43) Date of publication of application: **22.10.2025**
(21) Application number: 24170191.1
(22) Date of filing: 15.04.2024
(51) Int. Cl.: A61M 5/00

(54) **ASSEMBLY FOR PHARMACEUTICAL CONTAINERS FOR REDUCING CONTACT BETWEEN CONTAINERS AND SIDEWALLS OF ASSEMBLY**

(71) Applicant: SCHOTT Pharma Schweiz AG, 9001 St. Gallen (CH)
(72) Inventor: BERLINGER, Marcel, 9001 St. Gallen (CH); WALCHER, Ulrich, 9001 St. Gallen (CH)
(74) Representative: Schott Corporate IP

(57) **Abstract**

A structure comprising a plurality of receptacles, wherein
a. said receptacles are adapted and arranged for receiving a plurality of containers;
b. each receptacle, of the plurality of receptacles, comprises a sidewall defining an interior of a respective receptacle, wherein the sidewall comprises at least one protrusion extending into the interior of the respective receptacle;
wherein the at least one protrusion extends a first distance into the interior of the respective receptacle, wherein the first distance is in the range from > 0 to 1.5 mm.

## Description

### FIELD OF THE INVENTION

The invention pertains to a structure for transporting a plurality of containers, wherein said containers are preferably for accommodating pharmaceutical and/or cosmetic compositions. The invention also pertains to a use of the structure for transporting a plurality of containers.

### BACKGROUND

Containers, such as containers for pharmaceutical and/or cosmetic compositions, are generally received (e.g., held) and transported using structures known as nests, such as the nests commercially available from SCHOTT Pharma AG & Co. KGaA. These containers very often have a varying diameter. For example, the container may be a syringe with a cap, wherein a barrel of the syringe has a smaller diameter compared to a diameter of the cap. Therefore, the receptacles in the nest must be dimensioned such that containers with varying diameters can still be received in the nest. However, this leads to the containers tilting in the nest as there is very often a relatively large gap between the barrels and the receptacle sidewall. This further leads to contact between neighbouring containers received in the structure, which in turn causes damage to the containers. This contact between containers increases significantly if a nest has a high packaging density of the containers. Furthermore, contact between the container and the receptacle of the structure, wherein the container is received, can also lead to damage of the containers due to, e.g., the rubbing of the container against the sidewall of the receptacle. This is in particular problematic if there is contact between the container and a lower end of the receptacle. Structures for receiving and transporting containers are disclosed in US 2019070357 A and EP 2461849.

### OBJECTS

An object of the present invention is to at least partially overcome at least one of the disadvantages encountered in the state of the art.

It is a further object of the invention to provide a structure for receiving a plurality of containers, preferably for accommodating a pharmaceutical and/or cosmetic composition, wherein said structure reduces the contact between the containers and the sidewalls of the receptacles of the structure when said containers are received in said receptacles.

It is a further object of the invention to provide a structure for receiving a plurality of containers, preferably for accommodating a pharmaceutical and/or cosmetic composition, wherein said structure reduces the particle count on the surfaces of the containers when said containers are received in the structure.

It is a further object of the invention to provide a structure for receiving a plurality of containers, preferably for accommodating a pharmaceutical and/or cosmetic composition, wherein said structure reduces damage to the containers when said containers are inserted into, or removed from, the structure.

It is a further object of the invention to provide a structure for receiving a plurality of containers, preferably for accommodating a pharmaceutical and/or cosmetic composition, wherein said structure reduces damage to the containers when said containers are transported using the structure.

It is a further object of the invention to provide a structure for receiving a plurality of containers, preferably for accommodating a pharmaceutical and/or cosmetic composition, wherein damage to said structure is reduced when containers are inserted into, or removed from, the structure.

It is a further object of the invention to provide a structure for receiving a plurality of containers, preferably for accommodating a pharmaceutical and/or cosmetic composition, wherein said structure reduces the tilt of the containers when said containers are received in the structure.

It is a further object of the invention to provide a structure for receiving a plurality of containers, preferably for accommodating a pharmaceutical and/or cosmetic composition, wherein said structure can be used for containers that have a varying diameter (i.e., the diameter of the container varies, such as syringe with a cap wherein a barrel of the syringe has a smaller diameter than a diameter of the cap).

### PREFERRED EMBODIMENTS OF THE INVENTION

A contribution to at least partially fulfilling at least one of the above-mentioned objects is made by any of the embodiments of the invention.
A 1^{st} embodiment of the invention is a first structure comprising a plurality of receptacles, wherein
   a. said receptacles are adapted and arranged for receiving a plurality of containers, wherein said containers are preferably for accommodating pharmaceutical and/or cosmetic compositions;
   b. each receptacle, of the plurality of receptacles, comprises a sidewall defining an interior of a respective receptacle, wherein
      i. the sidewall comprises at least one protrusion extending into the interior of the respective receptacle,
      ii. preferably, the sidewall of the respective receptacle has at least one section that has a curved surface, and wherein the at least one section forms at least one protuberance that extends into the interior of the respective receptacle;
   wherein
   the at least one protrusion extends a first distance into the interior of the respective receptacle, wherein the first distance is in the range from > 0 to 1.5 mm, preferably from > 0 to 1 mm, more preferably from > 0 to 0.7 mm, and further preferably from > 0 to 0.5 mm.
   In a preferred aspect of the 1^{st} embodiment, the first distance is at least 0.01 mm, more preferably at least 0.05 mm, further preferably at least 0.1 mm. In a preferred aspect of the 1^{st} embodiment, the first distance is in the range from 0.01 to 1.5 mm, more preferably from 0.05 to 1 mm, further preferably from 0.1 to 0.5 mm.
A 2^{nd} embodiment of the invention is a further structure comprising a plurality of receptacles, wherein
   a. said receptacles are adapted and arranged for receiving a plurality of containers, wherein said containers are preferably for accommodating pharmaceutical and/or cosmetic compositions,
   b. each receptacle, of the plurality of receptacles, comprises a sidewall defining an interior of a respective receptacle, and wherein
      i. the sidewall has at least one section that has a curved surface, and wherein the at least one section forms at least one protuberance that extends into the interior of the respective receptacle,
      ii. preferably, the sidewall of the respective receptacle comprises at least one protrusion extending into the interior of the respective receptacle, and more preferably, wherein the at least one protrusion extends a first distance into the interior of the respective receptacle, wherein the first distance is in the range from > 0 to 1.5 mm, preferably from > 0 to 1 mm, more preferably from > 0 to 0.7 mm, and further preferably from > 0 to 0.5 mm.

In a preferred aspect of the 2^{nd} embodiment, the first distance is at least 0.01 mm, more preferably at least 0.05 mm, further preferably at least 0.1 mm. In a preferred aspect of the 2^{nd} embodiment, the first distance is in the range from 0.01 to 1.5 mm, more preferably from 0.05 to 1 mm, further preferably from 0.1 to 0.5 mm.

Preferred embodiments of "any of the structures" should be understood to be preferred embodiments of the first structure and the further structure. For example, the 3^{rd} to 25^{th} preferred embodiments of the invention are preferred embodiments of the first structure (of the 1^{st} embodiment of the invention) and the further structure (of the 2^{nd} embodiment of the invention).

In a preferred embodiment of any of the structures, the first distance is in the range from > 0 to 0.8 mm, more preferably from > 0 to 0.6 mm, even more preferably from > 0 to 0.4 mm, further preferably from > 0 to 0.3 mm, and even further preferably from > 0 to 0.2 mm.

This preferred embodiment is a 3^{rd} embodiment of the invention, that preferably depends on any of the 1^{st} to 2^{nd} embodiments of the invention. In a preferred aspect of the 3^{rd} embodiment, the first distance is at least 0.01 mm, more preferably at least 0.05 mm, and further preferably at least 0.1 mm. In a preferred aspect of the 3^{rd} embodiment, the first distance is in the range from 0.01 to 0.6 mm, more preferably from 0.05 to 0.4 mm, further preferably from 0.1 to 0.2 mm.

In a preferred embodiment of any of the structures, the at least one protrusion is arranged on the at least one protuberance.

This preferred embodiment is a 4^{th} embodiment of the invention, that preferably depends on any of the 1^{st} to 3^{rd} embodiments of the invention.

In a preferred embodiment of any of the structures, the at least one protrusion is adapted and arranged to create a gap between a first segment of the sidewall and a first segment of a container when said container is received (e.g., held) in the respective receptacle.

This preferred embodiment is a 5^{th} embodiment of the invention, that preferably depends on any of the 1^{st} to 4^{th} embodiments of the invention. In a preferred aspect of the 5^{th} embodiment, the at least one protrusion is adapted and arranged to create said gap at least at a further end of the respective receptacle.

In a preferred embodiment of any of the structures, the respective receptacle has a first end and a further end, and wherein the at least one protrusion is arranged closer to the further end than the first end.

This preferred embodiment is a 6^{th} embodiment of the invention, that preferably depends on any of the 1^{st} to 5^{th} embodiments of the invention.

In a preferred embodiment of any of the structures, the respective receptacle has a first end and a further end, and the at least one protrusion is arranged at a further distance of *xL* from the further end of the respective receptacle, where *L* is the length of the respective receptacle, and x is in the range from 0 to 0.25, preferably from 0 to 0.2, more preferably from 0 to 0.15, even more preferably from 0 to 0.1, and further preferably from 0 to 0.06.

This preferred embodiment is a 7^{th} embodiment of the invention, that preferably depends on any of the 1^{st} to 6^{th} embodiments of the invention. In a preferred aspect of the 7^{th} embodiment, x is in the range from 0.01 to 0.25, more preferably from 0.01 to 0.2, even more preferably from 0.01 to 0.15, further preferably from 0.02 to 0.1, and even further preferably from 0.03 to 0.06.

In a preferred embodiment of any of the structures, the respective receptacle has a first end and a further end, and the at least one protrusion is arranged at an even-further distance of *yL* from the further end of the respective receptacle, where *L* is the length of the respective receptacle, and *y* is in the range from 0.01 to 1, preferably from 0.02 to 0.7, more preferably from 0.03 to 0.5, even more preferably from 0.05 to 0.3, and further preferably from 0.07 to 0.1.

This preferred embodiment is an 8^{th} embodiment of the invention, that preferably depends on any of the 1^{st} to 7^{th} embodiments of the invention. In the 8^{th} embodiment *y* > *x*, where the at least one protrusion is arranged at a further distance of *xL* from the further end of the respective receptacle. In a preferred aspect of the 8^{th} embodiment, *y* is in the range from 0.01 to 0.3, more preferably from 0.01 to 0.2, even more preferably from 0.03 to 0.1, further preferably from 0.05 to 0.1, and even further preferably from 0.05 to 0.08.

In a preferred embodiment of any of the structures, a length of the at least one protrusion is in the range from 0.1 to 15 mm, preferably from 0.2 to 12 mm, even more preferably from 0.3 to 9 mm, and further preferably from 0.4 to 6 mm.

This preferred embodiment is a 9^{th} embodiment of the invention, that preferably depends on any of the 1^{st} to 8^{th} embodiments of the invention. In a preferred aspect of the 9^{th} embodiment, a length of the at least one protrusion is in the range from 0.05 to 3 mm, more preferably from 0.1 to 1.5 mm, even more preferably from 0.15 to 1 mm, and further preferably from 0.2 to 0.8 mm. In the 9^{th} embodiment, the length of a protrusion is measured along a direction that is parallel to a sidewall of the receptacle.

In a preferred embodiment of any of the structures, a surface area of the at least one protrusion is less than a surface area of the at least one protuberance.

This preferred embodiment is a 10^{th} embodiment of the invention, that preferably depends on any of the 1^{st} to 9^{th} embodiments of the invention.

In a preferred embodiment of any of the structures, the at least one protrusion covers a surface area of the sidewall of the respective receptacle that is 12 mm² or less, preferably 10 mm² or less, more preferably 7 mm² or less, even more preferably 5 mm² or less, and further preferably 3 mm² or less.

This preferred embodiment is a 11^{th} embodiment of the invention, that preferably depends on any of the 1^{st} to 10^{th} embodiments of the invention. In a preferred aspect of the 11^{th} embodiment, the surface area covered by the at least one protrusion is in the range from 0.03 to 12 mm², more preferably from 0.06 to 10 mm², more preferably from 0.08 to 7 mm², more preferably from 0.1 to 5 mm², more preferably from 0.13 to 3 mm², even more preferably from 0.16 to 1.5 mm², further preferably from 0.0.18 to 1 mm², and even further preferably from 0.21 to 0.7 mm².

In a preferred embodiment of any of the structures, a surface area of the at least one protrusion is in the range from 0.2 to 12 mm², preferably from 0.2 to 10 mm², more preferably from 0.2 to 8 mm², even more preferably from 0.3 to 6 mm², and further preferably from 0.3 to 4 mm². This preferred embodiment is a 12^{th} embodiment of the invention, that preferably depends on any of the 1^{st} to 11^{th} embodiments of the invention. In a preferred aspect of the 12^{th} embodiment, the surface area of the at least one protrusion is in the range from 0.2 to 2 mm², more preferably from 0.2 to 1 mm², even more preferably from 0.2 to 0.8 mm², and further preferably from 0.3 to 0.6 mm².

In a preferred embodiment of any of the structures, the respective receptacle comprises in the range from 1 to 10, preferably from 2 to 8, and further preferably from 4 to 6 protrusions.

This preferred embodiment is a 13^{th} embodiment of the invention, that preferably depends on any of the 1^{st} to 12^{th} embodiments of the invention.

In a preferred embodiment of any of the structures, at least one or all of the following applies to the at least one protrusion:
a. comprises at least one curved surface;
b. a shape of a cross-section of the at least protrusion is in the form of a circle segment or a circle;
c. a circumference of the at least one protrusion, measured at the base of the protrusion, is circular or elliptical.

This preferred embodiment is a 14^{th} embodiment of the invention, that preferably depends on any of the 1^{st} to 13^{th} embodiments of the invention. In an aspect of the 14^{th} embodiment, all possible combination of the features a. to c. are preferred aspects of the embodiment. These combinations are *e.g*., a; b; c; a+b; a+c; b+c; a+b+c. In the 14^{th} embodiment, the cross-sectional cut is made perpendicular to length of the respective receptacle. In the 14^{th} embodiment, a semicircle is an example of a circle segment. In the 14^{th} embodiment, the base of a protrusion is defined as the plane where the protrusion meets the sidewall (or the protuberance if present).

In a preferred embodiment of any of the structures, the at least one protuberance extends along at least 5 %, preferably at least 10 %, more preferably at least 20 %, and further preferably at least 30 % of a length of the respective receptacle.

This preferred embodiment is a 15^{th} embodiment of the invention, that preferably depends on any of the 1^{st} to 14^{th} embodiments of the invention. In a preferred aspect of the 15^{th} embodiment, the at least one protuberance extends along at least 50 %, preferably at least 70 %, more preferably at least 90 %, and further preferably at least 95 % of a length of the respective receptacle. In a preferred aspect of the 15^{th} embodiment, the at least one protuberance extends along 100 % of a length of the respective receptacle. In another preferred aspect of the 15^{th} embodiment, the at least one protuberance extends along less than 100 % of a length of the respective receptacle, e.g., 99 %.

In a preferred embodiment of any of the structures, a ratio of a minimum diameter to a maximum diameter of the respective receptacle is in the range from 1.01 to 2, preferably from 1.01 to 1.8, more preferably from 1.05 to 1.6, even more preferably from 1.05 to 1.4, and further preferably from 1.1 to 1.2, and wherein
a. the minimum diameter is measured between two points on the sidewall of the respective receptacle which are closest to each other, wherein a first imaginary line connecting these two closest points passes through a centre of the respective receptacle, and
b. the maximum diameter is measured between two points on the sidewall of the respective receptacle which are furthest from each other, wherein a second imaginary line connecting these two furthest points passes through a centre of the respective receptacle.

This preferred embodiment is a 16^{th} embodiment of the invention, that preferably depends on any of the 1^{st} to 15^{th} embodiments of the invention. In the 16^{th} embodiment, if a diameter of a respective receptacle varies along a length of the receptacle (e.g., the receptacle is narrower at the further end compared to the first end), the ratio in the 16^{th} embodiment is determined as follows: said ratio is determined at the first end of the receptacle to obtain a first ratio, and said ratio is determined at the further end of the receptacle to obtain a further ratio. The ratio of the minimum diameter to the maximum diameter of the respective receptacle is then calculated by taking the average of the first ratio and the further ratio.

In a preferred embodiment of any of the structures, a ratio of a width of the at least one protuberance to a minimum diameter of the respective receptacle is in the range from 0.1 to 0.95, preferably from 0.15 to 0.85, more preferably from 0.2 to 0.6, and further preferably from 0.3 to 0.4.

This preferred embodiment is a 17^{th} embodiment of the invention, that preferably depends on any of the 1^{st} to 16^{th} embodiments of the invention. In the 17^{th} embodiment, the minimum diameter is measured between two points on the sidewall of the respective receptacle which are closest to each other, wherein a first imaginary line connecting these two closest points passes through a centre of the respective receptacle. In the 17^{th} embodiment, the width is measured perpendicular to a further direction.

In a preferred embodiment of any of the structures, the respective receptacle comprises at least two protuberances, and wherein at least 10 %, preferably at least 30 %, more preferably at least 45 %, and further preferably at least 60 % of a circumference of the sidewall of the respective receptacle is in the form of the at least two protuberances.

This preferred embodiment is a 18^{th} embodiment of the invention, that preferably depends on any of the 1^{st} to 17^{th} embodiments of the invention. In a preferred aspect of the 18^{th} embodiment, in the range from 10 % to 100 %, preferably from 30 % to 80 %, more preferably from 45 % to 70 %, and further preferably from 50 % to 60 % of the circumference of the sidewall of the respective receptacle is in the form of the at least two protuberances.

In a preferred embodiment of any of the structures, a shape of a cross-section of the at least one protuberance is in the form of a circle segment or a circle.

This preferred embodiment is a 19^{th} embodiment of the invention, that preferably depends on any of the 1^{st} to 18^{th} embodiments of the invention. In the 19^{th} embodiment, the cross-sectional cut is made perpendicular to length of the respective receptacle. In the 19^{th} embodiment, a semicircle is an example of a circle segment.

In a preferred embodiment of any of the structures, at least one or all of the following applies:
a. the at least one protuberance is filled;
b. the at least one protuberance is hollow.

This preferred embodiment is a 20^{th} embodiment of the invention, that preferably depends on any of the 1^{st} to 19^{th} embodiments of the invention. In an aspect of the 20^{th} embodiment, all possible combination of the features a. and b. are preferred aspects of the embodiment. These combinations are *e.g*., a; b; a+b. Regarding the combination a+b: this should be understood to mean that there is a plurality of protuberances, with some of these protuberances at least partially filled, and some of these protuberances being hollow.

In a preferred embodiment of any of the structures, a diameter of the interior varies along a length of the respective receptacle, preferably wherein a first diameter of the interior, measured at a first end of the respective receptacle, is larger than a further diameter, measured at a further end of the respective receptacle.

This preferred embodiment is a 21^{st} embodiment of the invention, that preferably depends on any of the 1^{st} to 20^{th} embodiments of the invention.

In a preferred embodiment of any of the structures, the plurality of receptacles are adapted and arranged to elastically deform.

This preferred embodiment is a 22^{nd} embodiment of the invention, that preferably depends on any of the 1^{st} to 21^{st} embodiments of the invention. An example of the elastic deformation in the 22^{nd} embodiment is the following: a plurality of containers is received in the plurality of receptacles. During transport of said containers, the containers move, and the plurality of receptacles elastically deform.

In a preferred embodiment of any of the structures, at least a portion of a sidewall of at least one receptacle does not touch the sidewall of at least one neighbouring receptacle.

This preferred embodiment is a 23^{rd} embodiment of the invention, that preferably depends on any of the 1^{st} to 22^{nd} embodiments of the invention. An example of the 23^{rd} embodiment is gaps or spaces that are formed between portions of the sidewalls, of neighbouring receptacles, that do not touch.

In a preferred embodiment of any of the structures, the respective receptacle comprises at least two, preferably at least three, more preferably at least four, and further preferably at least five protuberances.

This preferred embodiment is a 24^{th} embodiment of the invention, that preferably depends on any of the 1^{st} to 23^{rd} embodiments of the invention. In a preferred aspect of the 24^{th} embodiment, the structure comprises in the range from 2 to 10, preferably from 3 to 8, more preferably from 4 to 8, and further preferably from 4 to 6 protuberances.

In a preferred embodiment of any of the structures, a further segment of the respective receptacle is adapted and arranged to receive a further segment of the container, wherein the further segment of the containers has a dimension that is larger than the average diameter of the container. This preferred embodiment is a 25^{th} embodiment of the invention, that preferably depends on any of the 1^{st} to 24^{th} embodiments of the invention. In a preferred aspect of the 25^{th} embodiment, the further segment of the respective receptacle is located at the first end of the receptacle. In a preferred aspect of the 25^{th} embodiment, the further segment of the container is located at an end of the container. An example of the further segment of the container is a flange of syringe.

Embodiments and preferred embodiments employing "a structure" and/or using "any of the structures" should be understood to be embodiments and preferred embodiments employing and/or using the first structure, the further structure, or both.

A 26^{th} embodiment of the invention is a method for transporting a plurality of containers, comprising the steps of
a. providing a structure according to the invention (e.g., the first structure, the further structure), wherein said structure is preferably a structure according to any of the 1^{st} to 25^{th} embodiments of the invention;
b. receiving a plurality of containers in the structure;
c. transporting the structure and the plurality of containers from a first location to a further location.

In a preferred embodiment of the method for transporting a plurality of containers, the method further comprises at least one or all of the following steps:
A. inserting the structure into an even-further structure that at least partially encloses the structure, e.g., a tub;
B. sealing an opening of the even-further structure with a film, preferably a gas permeable film;
C. sterilising the structure and the containers received therein, preferably while sealed in an even-further structure, e.g., the tub.

This preferred embodiment is a 27^{th} embodiment of the invention, that preferably depends on the 26^{th} embodiment of the invention. In an aspect of the 27^{th} embodiment, all possible combination of the features A. to C. are preferred aspects of the embodiment. These combinations are *e.g.,* A; B; C; A+B; A+C; B+C; A+B+C. In the 27^{th} embodiment, a "tub" should be understood as used in the pharmaceutical industry. In a preferred aspect of the 27^{th} embodiment, one or more of the steps A. to C. are performed at the first location, while one or more of the steps A. to C. are performed at the further location. For example, steps A. and B. are performed at the first location after performing step b. in the 26^{th} embodiment, the structure with the containers are then transported to the further location (step c. in 26^{th} embodiment), where step C. is then performed at the further location.

A 28^{th} embodiment of the invention is a use of any of the structures according to the invention, preferably any of the structure according to any of the 1^{st} to 25^{th} embodiments of the invention, for the transport of a plurality of containers received in said structure.

A 29^{th} embodiment of the invention is a use of any of the structures according to the invention, preferably any of the structure according to any of the 1^{st} to 25^{th} embodiments of the invention, for at least one or all of the following:
a. reducing contact between the further ends of the plurality of receptacles and the plurality of containers received in said receptacles;
b. reducing contact between neighbouring containers received in the structure;
c. reducing a particle count on the surfaces of the containers received in the structure;
d. reducing damage to the containers when received in the structure.

In an aspect of the 29^{th} embodiment, all possible combination of the features a. to d. are preferred aspects of the embodiment. These combinations are *e.g.,* a; b; c; d; a+b; a+c; a+d; b+c; b+d; c+d; a+b+c; a+b+d; a+c+d; b+c+d; a+b+c+d. In the 29^{th} embodiment, the particle count is reduced the interior surfaces of the containers, or the exterior surfaces of the containers, or both.

### DETAILED DESCRIPTION OF THE INVENTION

Features described as preferred in one category of the invention, for example according to the structure, are analogously preferred in an embodiment of the other categories according to the invention, such as a use of the structure according to the invention.

Throughout this document, disclosures of ranges should preferably be understood to include both end points of the range. Furthermore, each disclosure of a range in the document should preferably be understood as also disclosing preferred sub-ranges in which one end point is excluded or both end points are excluded. For example, a disclosure of a range from X₁ to X₂ is to be understood as disclosing a range that includes both of the end points Xi and X₂. Further-more, it is to be understood as also disclosing a range that includes the end point Xi but excludes the end point X₂, a range that excludes the end point Xi but includes the end point X₂, and a range that excludes both end points Xi and X₂.

Some preferred embodiments and preferred aspects have various combinations of features as alternatives. If the various combinations are disclosed, these combinations are separated by a semi-colon (";"). For example, the list of features "a; a+b; a+c+d" for a preferred embodiment discloses a preferred embodiment that comprises the feature "a", a preferred embodiment that comprises the features "a" and "b", and a preferred embodiment that comprises the features "a", "c", and "d".

Preferred embodiments and preferred aspects disclosed for a structure should be understood to be preferred embodiments and preferred aspects of both the first structure and the further structure.

In one embodiment of the invention, the structure comprises a plurality of receptacles, wherein each receptacle, of the plurality of receptacles, has a sidewall that has at least one section that has a curved surface, and wherein the at least one section forms a protuberance. This feature is referred herein as "the sidewall comprises a protuberance".

In an embodiment of the invention, the structure comprises a plurality of receptacles. Each receptacle, of the plurality of receptacles, has a sidewall comprising at least one protrusion and/or at least one protuberance. This should not be understood to mean that the structure can only have receptacles with sidewalls that comprise at least one protrusion and/or at least one protuberance. The structure may, in addition to the aforementioned plurality of receptacles, e.g., further comprise a further plurality of receptacles, wherein the sidewalls of the receptacles (of the further plurality of receptacles) comprises neither a protrusion nor a protuberance.

In an aspect of the invention, it is preferred that at least 70 %, more preferably at least 85 %, even more preferably at least 95 %, and further preferably all of the receptacles of the first structure form part of the plurality of receptacles (i.e., having sidewalls comprising at least one protrusion and/or at least one protuberance).

Preferred embodiment and preferred aspects of "the respective receptacle" should be understood to be preferred embodiment and preferred aspects of at least 70 %, more preferably at least 85 %, even more preferably at least 95 %, and further preferably all of the receptacles that form the plurality of receptacles.

Embodiments and preferred embodiments of the at least one protrusion should preferably be understood to apply separately to each of the protrusions of "the at least one protrusion". For example, if a structure (e.g., the first structure, the further structure) comprises at least one protrusion that extends a first distance of 0.2 mm into the receptacle, this should preferably be understood to mean that the structure has one or more protrusions, wherein each of these protrusions extends a first distance of 0.2 mm into the receptacle. For example, if a structure (e.g., the first structure, the further structure) comprises at least one protrusion that covers a surface area of 1.5 mm² of a sidewall of a receptacle, this should preferably be understood to mean that the structure has one or more protrusions, wherein each of these protrusions covers a surface area of 1.5 mm² of a sidewall of a receptacle.

Embodiments and preferred embodiments of the at least one protuberance should preferably be understood to apply separately to each of the protuberances of "the at least one protuberance. For example, if a structure (preferably the first structure, the further structure) comprises at least one protuberance that extends along at least 50 % a length of the respective receptacle, this should preferably be understood to mean that the structure has one or more protuberances, wherein each of these protuberances extends along at least 50 % of the length of the respective receptacle.

A container that is received in a receptacle should preferably be understood to mean that the container is held in said receptacle.

### Structure

A structure (e.g., the first structure, the further structure) preferably comprises a polymer. Examples of a preferred polymer include polypropylene, polyoxymethylene, polyethylene, polyethylene terephthalate, polystyrene, a cyclic olefin copolymer (COC) and a cyclic olefin polymer (COP). Here polypropylene is particularly preferred for the structure.

A structure may be obtained using deep drawing moulding, preferably injection moulding, and/or 3D printing. A structure is preferably manufactured as a single piece. A structure for receiving containers, wherein said containers are for accommodating pharmaceutical and/or cosmetic compositions, is often referred to as a "nest". Here "nest" should preferably be understood as used in the pharmaceutical industry.

A structure preferably comprises 5 to 250, more preferably 10 to 200, even more preferably 20 to 190, further preferably 40 to 180, further preferably 60 to 180, further preferably 80 to 180, and even further preferably 100 to 160, receptacles.

### Receptacle

A receptacle preferably extends from a first surface of the structure. A preferred receptacle is elongated. A preferred receptacle has a first end and a further end, opposite the first end. A first end of a receptacle, of the plurality of receptacles, preferably has a first opening for receiving a container. In other words, a receptacle, of the first plurality of receptacles, is preferably adapted and arranged for a container to be inserted into and/or removed from said respective receptacle at the first end. Preferably, the further end of a receptacle, of the plurality of receptacles, has a further opening. A further opening at the further end of a receptacle, of the plurality of receptacles, is preferably adapted and arranged to allow a part of a container to extend out of the receptacle when said container is received in the receptacle.

A receptacle that is adapted and arranged to elastically deform should preferably be understood as follows. The receptacle has a first shape. Upon application of a force, the receptacle (e.g., the sidewalls) deforms to have a further shape. Upon removal of the force, the receptacle at least partially returns to having the first shape.

### Protrusions and protuberances

A protrusion preferably comprises a polymer. Examples of a preferred polymer include polypropylene, polyoxymethylene, polyethylene, polyethylene terephthalate, polystyrene, a cyclic olefin copolymer (COC) and a cyclic olefin polymer (COP). Here polypropylene is particularly preferred for a protrusion. A protrusion is preferably formed as one piece with the sidewall of the receptacle, of the plurality of receptacles. In a preferred aspect of the invention, a protrusion extends in a direction that is substantially perpendicular to the sidewall of the receptacle.

A protuberance preferably comprises a polymer. Examples of a preferred polymer include polypropylene, polyoxymethylene, polyethylene, polyethylene terephthalate, polystyrene, a cyclic olefin copolymer (COC) and a cyclic olefin polymer (COP). Here polypropylene is particularly preferred for a protuberance. A protuberance is preferably formed as one piece with the sidewall of the receptacle, of the plurality of receptacles. In a preferred aspect of the invention, a protuberance extends in a direction that is substantially perpendicular to the sidewall of the receptacle.

The feature "the sidewall of the respective receptacle has at least one section that has a curved surface, wherein the at least one section forms at least one protuberance" should be understood to mean that the protuberance has a curved surface. A curvature, of a surface of a section of the sidewall forming a protuberance, is preferably perpendicular to a length of a respective receptacle.

### Protrusions arranged on protuberances

In a preferred embodiment of the invention, the at least one protrusion is arranged on the at least one protuberance. This embodiment should not be understood to imply that the number of protrusions is necessarily equal to the number of protuberances. The number of protrusions may be more than, equal to, or less than, the number of protuberances. The preferred embodiment should rather be understood to mean that each protrusion is arranged on a protuberance. For example, a respective receptacle of a structure has four protrusions and two protuberances, wherein two protrusions are arranged on each of the two protuberances. For example, a respective receptacle of a structure has four protrusions and four protuberances, wherein one protrusion is arranged on each of the four protuberances. For example, a respective receptacle of a structure has two protrusions and four protuberances, wherein one protrusion is arranged on two of the protuberances, while the remaining protuberances do not have a protrusion.

### Containers

A container as referred herein comprises a container wall at least partially enclosing an interior volume of said container. A container as referred to herein is designed to accommodate a composition in such a way that an inner side of the container wall (i.e., the side of the container wall which faces the interior volume) and/or a coating layer (e.g., lubrication layer) on the inner side of the container wall can be in direct contact with the composition. A preferred container is suitable to be used for accommodating a pharmaceutical and/or cosmetic composition.

A first end of a container preferably comprises a first orifice, which allows for the charging of a composition into the interior volume of the container. Preferably, the first orifice also allows for the discharging of a composition from the interior volume.

An interior volume of a container represents the full volume of the interior of the container. This volume may be determined by filling the interior volume with water up to the brim of the container and measuring the volume of the amount of water which the interior volume can take up to the brim. Hence, the interior volume as used herein is not a nominal volume as it is often referred to in the technical field of pharmacy. This nominal volume may for example be less than the interior volume. Fill volume should preferably be understood as the volume of a composition in a container.

A container according to the invention may have any size or shape which the skilled person deems appropriate in the context of the invention. A preferred container has at least one section that is cylindrical. A preferred container is elongated.

A preferred container, more preferably the container wall, comprises, more preferably consists of, at least one glass, at least one polymer, or a combination thereof. A glass may be any type of glass and may have any composition which the skilled person deems suitable in the context of the invention. Preferably, the glass is suitable for pharmaceutical compositions. A preferred glass is of type I in accordance with the definitions of glass types in section 3.2.1 of the European Pharmacopoeia, 7th edition from 2011. Examples of a preferred glass include borosilicate glass, fused silica, and a combination thereof. A particularly preferred glass is a borosilicate glass. Examples of a preferred polymer include polypropylene, polyoxymethylene, polyethylene, a cyclic olefin copolymer (COC) and a cyclic olefin polymer (COP). Here a cyclic olefin copolymer (COC) and a cyclic olefin polymer (COP) are particularly preferred.

Examples of preferred containers include cartridges, vials, ampules, carpules, and syringes. Preferably, all of the containers, of the plurality of containers, are of the same type of container, which is preferably selected from the above-mentioned mentioned examples of preferred containers. A preferred syringe is made from glass or polymer.

Additionally or alternatively, a preferred syringe has an interior volume in the range from 0.5 to 100 ml, preferably from 0.5 to 80 ml, more preferably from 0.5 to 60 ml. Additionally or alternatively, a preferred syringe comprises a body part which is preferably cylindrical. This is also referred to as barrel. A preferred barrel of a syringe has a diameter in the range from 5 to 50 mm, more preferably from 6 to 45 mm, even more preferably from 8 to 40 mm, most preferably from 9 to 35 mm. Particularly preferable, the barrel is made from a glass or a polymer.

A preferred container, more preferably the container wall, more preferably a surface of the container wall facing the interior volume of the container, comprises at least one coating layer. Here a coating layer should preferably be understood as a layer that is applied by any method known to a skilled person working in the pharmaceutical industry. These methods include the application of a coating layer by spraying or wiping said coating layer on the container wall facing the interior volume of the container. A preferred coating layer is a lubrication layer. A preferred lubrication layer comprises silicon. A preferred lubrication layer reduces friction when a component of the container is moved in the interior volume of the container. Here an example of a component is a plunger of a syringe, with the syringe being an example of a container. For example, a lubrication layer reduces the force required to move a plunger in a barrel of a syringe.

A preferred containers has a first section and a further section, wherein a diameter of the further section is larger than a diameter of the first section. Here it is preferred that the further section is a body of the container, and the further section is a closing means adapted and arranged to close the container. For example, the first section is a barrel of a syringe, and the further section is a cap of the syringe.

### Pharmaceutical and cosmetic composition

In the context of the invention, every pharmaceutical composition and every cosmetic composition which the skilled person deems suitable comes into consideration. A pharmaceutical composition is a composition comprising at least one pharmaceutically active ingredient. A preferred pharmaceutically active ingredient is a vaccine. A cosmetic composition is a composition comprising at least one cosmetically active ingredient. A preferred cosmetically active ingredient is hyaluronic acid or botulinum toxin. The pharmaceutical and/or cosmetic composition may be fluid or solid or both, wherein a fluid composition is particularly preferred herein. A preferred solid composition is granular such as a powder, a multitude of tablets or a multitude of capsules. A further preferred pharmaceutical and/or cosmetic composition is a parenterialium, i.e., a composition which is intended to be administered via the parenteral route, which may be any route which is not enteral. Parenteral administration can be performed by injection, e.g., using a needle (usually a hypodermic needle) and a syringe, or by the insertion of an indwelling catheter.

The invention is now illustrated by non-limiting examples and exemplifying embodiments.

### FIGURES

### List of figures

The figures serve to exemplify the present invention and should not be viewed as limiting the invention. Furthermore, the figures are not drawn to scale.
Fig. 1: schematic illustration of a cross-section of a structure according to the invention.
Fig. 2: schematic illustration showing variations of a receptacle, of the plurality of receptacles.
Figs 3A to 3E: illustration of definitions of a protrusion.
Fig. 4: isometric view of a structure according to the invention.
Fig. 5: cross-sectional cut of receptacles of a structure according to the invention.
Fig. 6: perpendicular cross-sectional cut of receptacles, of the plurality of receptacles.
Fig. 7: flow diagram showing the steps of a method for transporting a plurality of containers.
Figs 8A to 8D: schematic illustrations showing alternative embodiments of a protrusion.
Figs 9A and 9B: cross-section of receptacles used in simulation.
Fig. 10: graphs showing simulation results.

### Description of figures

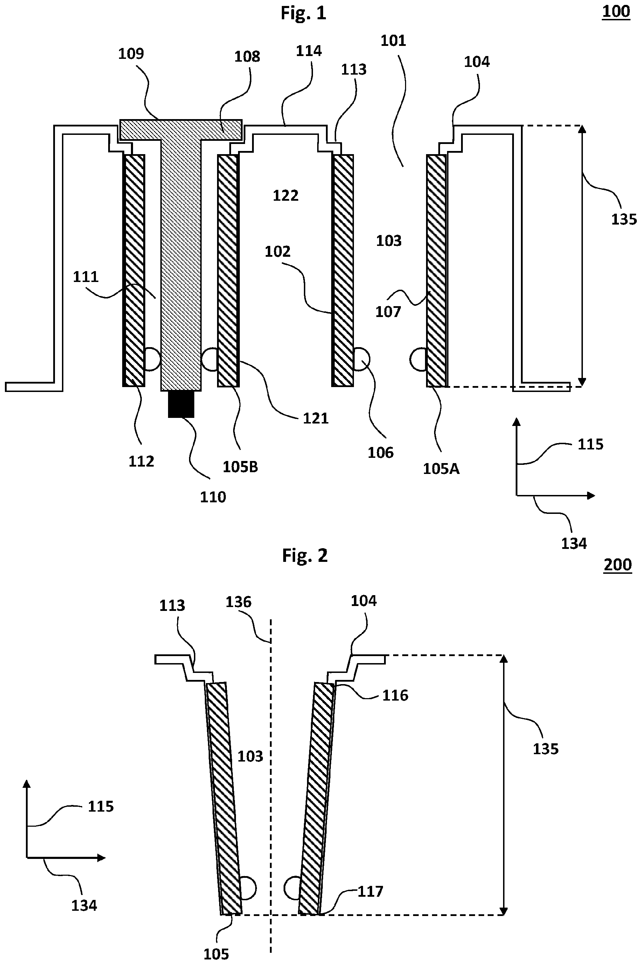

Fig. 1 shows a schematic illustration of a cross-section of a structure 100 according to the invention. The structure 100 comprises a plurality of receptacles, such as receptacle 101 (for the purposes of illustration, only two receptacles are shown). The receptacles, of the plurality of receptacles, will be described using receptacle 101 as an example. The receptacle 101 has a sidewall 102 defining an interior 103 of the receptacle 101. The receptacle 101 is elongated with a first end 104 and a further end 105A. The sidewall 102 comprises protrusions, such as protrusion 106, and protuberances, such as protuberance 107 that extend into the interior 103. The protrusions and protuberances are formed as one piece with the sidewalls of the receptacles. Fig. 1 also shows that the protuberance 107 extends along a length of the receptacle 101. However, protuberance 107 does not extend along the entire length of the receptacle 101. The receptacle 101 is adapted and arranged for receiving a container, such as container 108. The container 108 is for accommodating a pharmaceutical composition. The container 108 has a first end 109 and a further end 110. When a container is received in a receptacle, of the plurality of receptacles, a gap is formed between a first segment of the container and a first segment of the sidewall of said receptacle. An example is the gap 111 shown in Fig. 1 formed between the container 108 and the protuberance 112 (protuberance 112 forms part of the sidewall). This gap 111 is formed due to the presence of the protrusions (but may also be formed due to the presence of the protuberances). It is more preferred that the gap 111 is formed at least at the further end 105B of the receptacle 101, thereby reducing the contact between the container 108 and the further end 105B of the receptacle holding the container (the further ends of the receptacles very often have sharp edges that in particular damages the containers when the containers come into contact with said edges). A gap at the further end of a receptacle is in particular preferred if the receptacle becomes narrower towards the further end of said receptacle (see Fig. 2).

As can be seen from Fig. 1, the container 108 is a syringe with a flange. I.e., the first end 109 (the flange) has a dimension that larger than an average diameter of the container 108. The receptacle 101 is correspondingly adapted and arranged to have a further segment 113 that is adapted and arranged to receive the flange of a syringe. Preferably, the further segment 113 is dimensioned such that when a container is received in the receptacle 101, only a part of said container extends above a first surface 114 of the structure 100. Alternatively, the further segment 113 may be dimensioned such that when a container is received in the receptacle 101, no part of said container extends above a first surface 114 of the structure 100 (this includes a top of the further segment 113 being level with, or below, the first surface 114).The length 135 of receptacle 101 is defined as the distance between the first end 104 and the further end 105, as measured along a further direction 115. In Fig. 1, the further direction 115 is perpendicular to both the first surface 114 and a first direction 134. Fig. 1 also shows that the portions of the sidewalls 121 and 102 of the neighbouring receptacles do not teach each other, thereby forming a space or gap 122 between the neighbouring receptacles. This allows the receptacles to elastically deform during insertion and removal of containers from the receptacles.

Fig. 2 is a schematic illustration showing variations of a receptacle 200, of the plurality of receptacles. A diameter of the receptacle 200, measured at point a 116, is larger than a diameter of the receptacle 200 measured at a point 117. The point 116 is located closer to the first end 104 than the further end 105 of the receptacle 200. The point 117 is located at the further end 105. The receptacle 101 thus becomes narrower towards the further end 105. Fig. 2 also shows that the receptacle 200 has a further segment 113 for receiving, e.g., a flange of a syringe. As can also be seen in Fig. 2, the sidewall of the further segment 113 is slanted towards an interior 103 of the receptacle 200. It should be noted that having a receptacle with a diameter that varies along the length of the receptacle does not require the further segment, such as the further segment 113. If the receptacle has a further segment, it is not required that the sidewall of the further segment is slanted. The length 135 of the receptacle 200 is also measured along the further direction 115 between the first end 104 and the further end 105 of the receptacle. This further direction 115 is parallel to a further imaginary line 136 that passes through a centre of the receptacle 200.

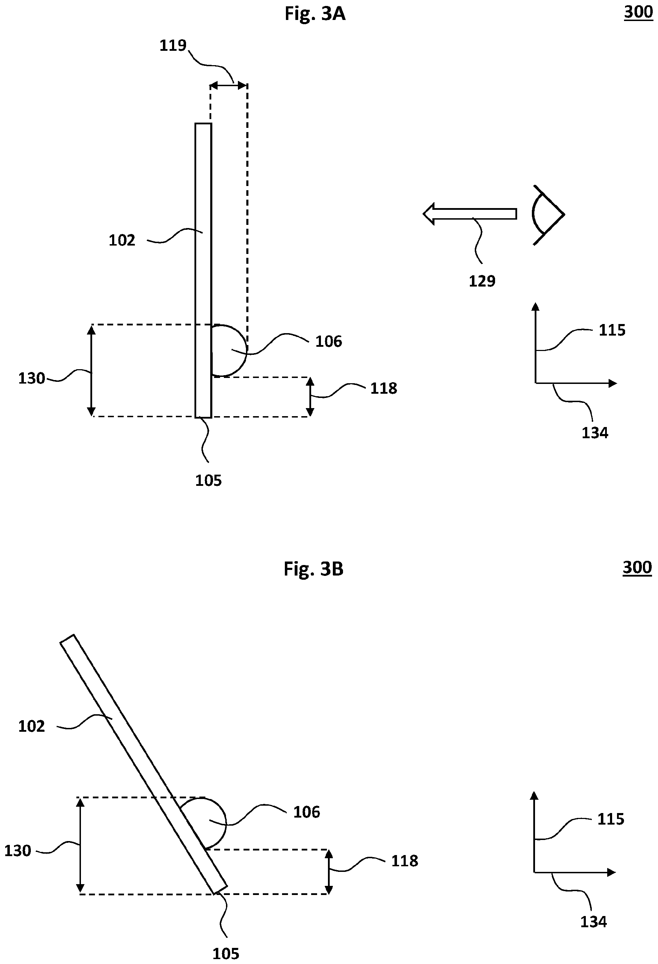

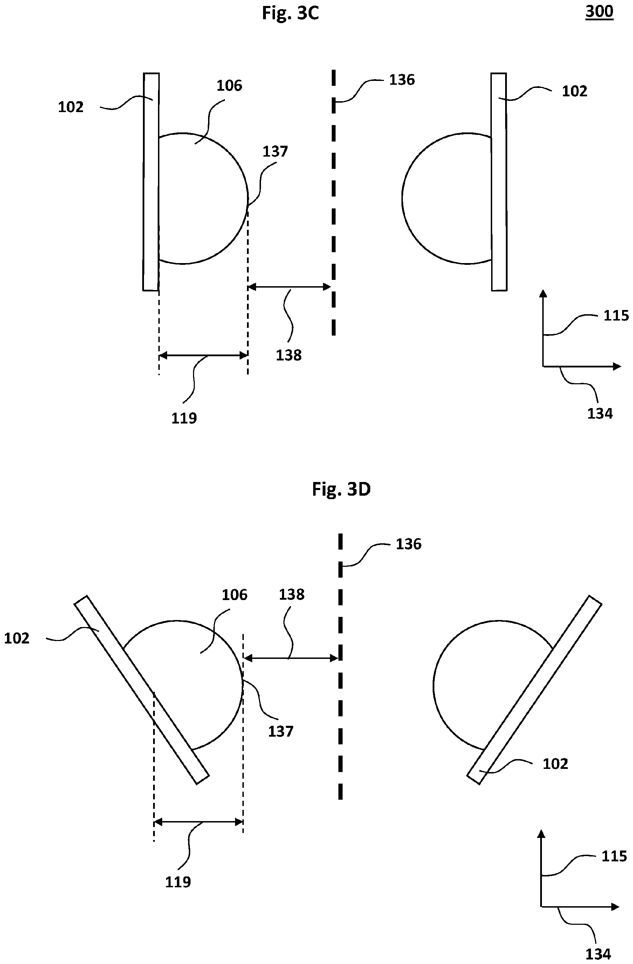

Figs 3A to 3E illustrate how the dimensions of a protrusion are measured. Fig. 3A is a schematic illustration showing a cross-section of a protrusion 106 and a part of a receptacle. The cross-section is made along the length of a receptacle, i.e., Fig. 3A is a sideview of the protrusion 106. The protrusion 106 is arranged at a further distance 118 of *xL* from the further end 105, where *L* is the length of the receptacle where the protrusion 106 is arranged. The further distance 118 is measured from the further end 105 to the point of the protrusion 106 closest to the further end 105. A further distance where *x* = 0 indicates that the point on the protrusion closest to the further end of the receptacle is arranged flush with the further end 105, while a further distance where *x* = 0.5 indicates that the point on the protrusion closest to the further end of a receptacle is arranged at the middle of the receptacle. Further 0 ≤ *x* < 1. The protrusion 106 is also arranged at an even-further distance 130 of *yL* from the further end 105 of the receptacle. The even-further distance 130 is measured from the further end 105 to the point on the protrusion 106 furthest from the further end 105. A value of *y* = 1 indicated that the point of the protrusion furthest from the further end of the receptacle is arranged flush with the first end of the receptacle. The further distance 118 and the even-further distance 130 are measured along the further direction 115, wherein said direction is parallel to a further imaginary line that passes through the centre of the receptacle (see Fig. 3C).

Fig. 3A also shows how the first distance 119 of the protrusion 106 is measured. The first distance 119 in Fig. 3A is measured from the sidewall 102 to the point of the protrusion 106 closest to a further imaginary line passing through a centre of the receptacle (see further imaginary line 136 in Fig. 2). If the protrusion 106 is arranged on a protuberance, the distance is measured from the protuberance to the point of the protrusion closest to the further imaginary line. The first distance 119 is measured along the first direction 134.

Fig. 3B is similar to Fig. 3A, with the following difference. The sidewall 102 of the receptacle in Fig. 3B is arranged at an angle with respect to the further direction 115 (in Fig. 3A the sidewall is arranged parallel to the further direction). The further distance 118 and the even-further distance 130 are measured as shown in Fig. 3B, i.e., parallel to the further direction 115 (and not along a direction that is parallel to the sidewall 102).

The measurement of the first distance 119 is further illustrated in Fig 3C and 3D. Figs 3C and 3D are schematic illustration showing cross-sections of a protrusion 106 and a part of a receptacle. Fig. 3C shows part of a receptacle with sidewalls 102 that are parallel to the further direction 115. A further imaginary line 136 passes through the centre of the receptacle. The further imaginary line 136 is also parallel to the further direction 115. Point 137 on the protrusion 106 is the point on said protrusion that is closest to the further imaginary line 136 (indicated by the distance 138) when measured along the first direction 134. The first distance 119 is measured between the sidewall 102 and the point 137 along the first direction 134. Fig. 3D shows part of receptacle with sidewalls 102 that are arranged at an angle to the further direction 115. A further imaginary line 136 passes through the centre of the receptacle (see also Fig. 2). The further imaginary line 136 is also parallel to the further direction 115. Point 137 on the protrusion 106 is the point on said protrusion that is closest to the further imaginary line 136 (indicated by the distance 138) when measured along the first direction 134. The first distance 119 is measured between the sidewall 102 and the point 137 along the first direction 134.

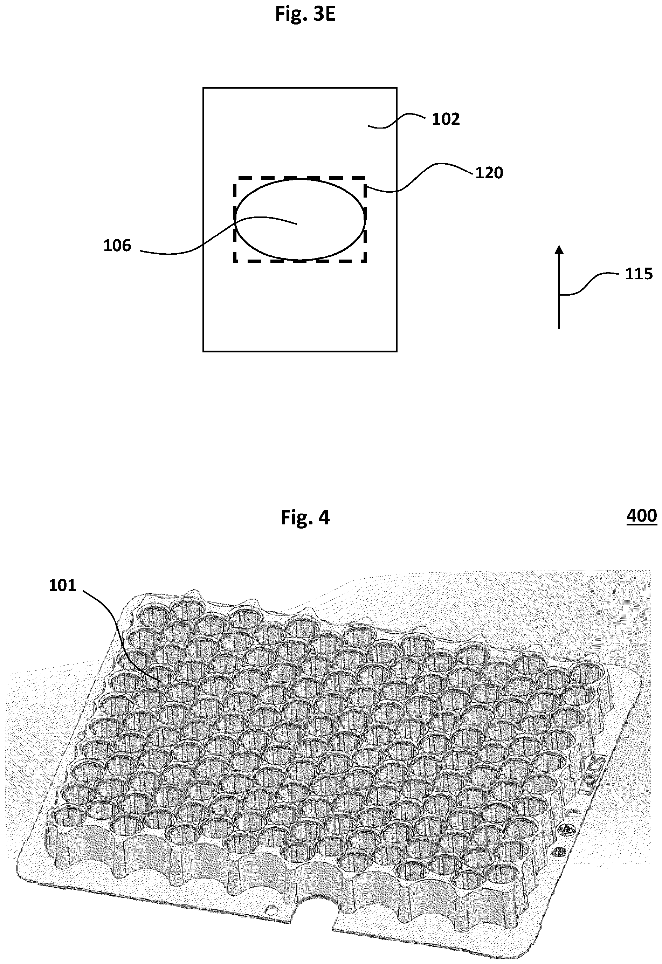

Fig. 3E illustrates how a surface area of a sidewall, covered by a protrusion, is determined. Fig. 3E is a schematic illustration showing a protrusion 106 viewed from the front (i.e., in the direction 129 indicated in Fig. 3A, perpendicular to the sidewall 102). The protrusion 106 is arranged on the sidewall 102. An imaginary rectangle 120 is drawn around the protrusion 106 (a square is an example of a rectangle). The rectangle 120 is chosen as the smallest rectangle that encloses all points of the protrusion 106 (i.e., the rectangle with the smallest surface area). The surface area of the rectangle 120 is defined as the surface area of a sidewall 102 covered by a protrusion 106. It should be noted that the surface area of a protrusion and the surface area of a sidewall, covered by the protrusion, are two different features. For example, a protrusion that is in the form of a half-sphere has a surface area of 2π*r*², where *r* is the radius of the protrusion. This half-sphere protrusion will cover a surface area of the sidewall that is 4*r*² (the rectangle 120 enclosing the protrusion will be a square with sides 2*r*).

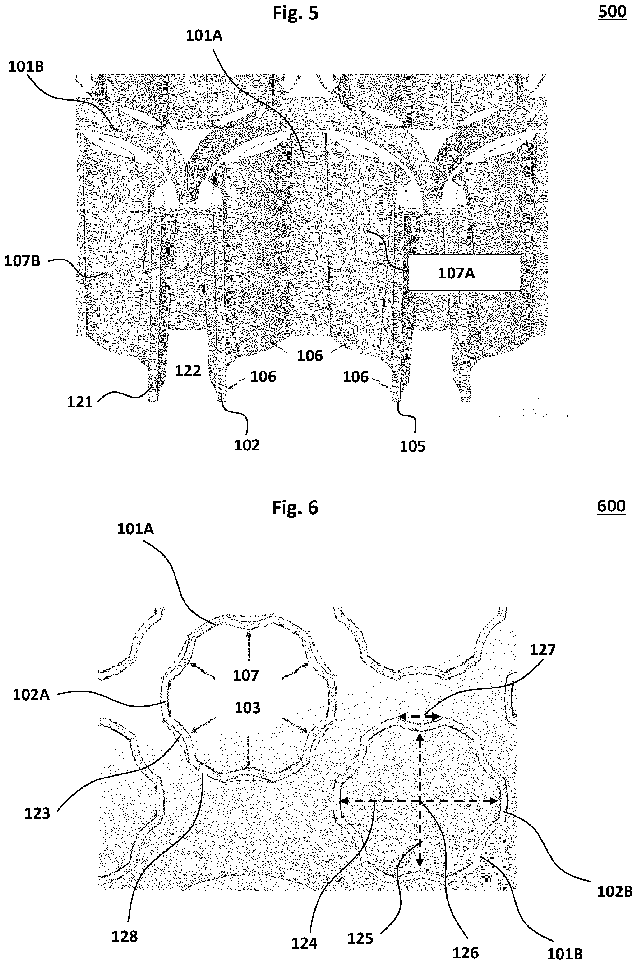

Fig. 4 is an isometric view of a structure 400 according to the invention, wherein the structure 400 comprises a plurality of receptacles, such as receptacle 101. Fig. 5 shows a cross-sectional cut of receptacles of a structure according to the invention. The cross-section cut is made parallel to the length of the receptacles. Receptacle 101A has a sidewall 102 that has a number of sections with curved surfaces. These sections form protuberances, such as protuberance 107A, that extend into the interior of receptacle 101A (the neighbouring receptacle 101B has, e.g., protuberance 107B). The protuberances extend almost along the entire length of the receptacle 101A. The protuberances are also formed as one piece with the sidewall 102, as well as being filled. Arranged on the protuberances are the protrusions 106 (indicated by the arrows) that also extend into the interior of the receptacle 101A. The protrusions 106 are arranged on protuberances such that each protrusion is arranged on a different protuberance. It can also be seen from Fig. 5 that the protrusions 106 are oval shaped, i.e., the circumference of a base of a protrusion is elliptical (the base of the protrusion is where the protrusions meet the protuberances). Fig. 5 further shows that portions of the sidewalls 102 and 121 of neighbouring receptacles 101A and 101B, respectively, do not touch, thereby forming a gap or space 122.

Fig. 6 shows a cross-section cut of receptacles, of the plurality of receptacles, made perpendicular to a length of the receptacles. Fig. 6 shows that a sidewall 102A of receptacle 101A has a number of sections, such as section 123, that have curved surfaces. These sections with curved surfaces form protuberances 107 (indicated by the arrows) that extend into the interior 103 of receptacle 101A.

Fig. 6 also shows how a maximum and a minimum diameter of a receptacle is measured. As shown for receptacle 101B, the maximum diameter is measured between two points on the sidewall 102B that are furthest from each other, wherein a first imaginary 124 line connecting these two furthest points passes through a centre 126 of receptacle 101B. The length of the first imaginary line 124 is the maximum diameter. The minimum diameter is measured between two points on the sidewall 102B that are closest to each other, wherein a second imaginary line 125 connecting these two closest points passes through the centre 126 of receptacle 101B. The length of the second imaginary line 125 is the minimum diameter. In Fig. 6, the minimum diameter is measured between two protuberances. If the sidewall has protrusions that are arranged on the protuberances, the two closest points may be on two protrusions arranged opposite each other on the sidewall. Fig. 6 also shows how a width 127 of a protuberance is measured.

Fig. 6 also shows that at least 50 %, but less than 100 % of a circumference of the receptacles 101A and 101B are in the form of protuberances. The receptacle 101A has sections, such as section 128, that do not have curved surfaces that form protuberances that extend into the interior 103. It should however be noted that the section 128 may have a curved surface that extends away from the interior 103.

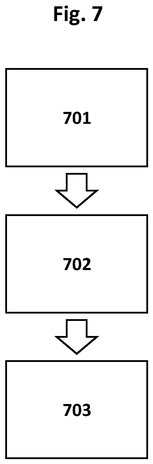

Fig. 7 is a flow diagram showing the steps of a method for transporting a plurality of containers. In step 701, a structure according to the invention is provided. In step 702, a plurality of containers is received in the structure. In step 703, the structure and the plurality of containers are transported from a first location to a further location.

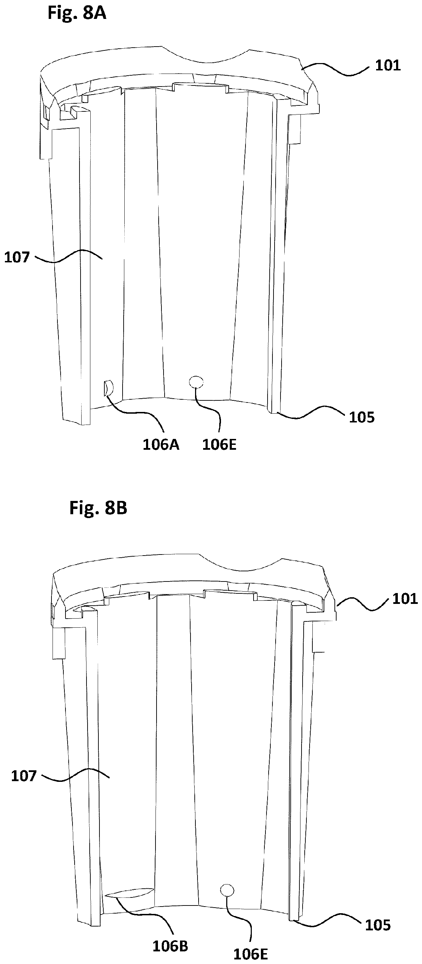

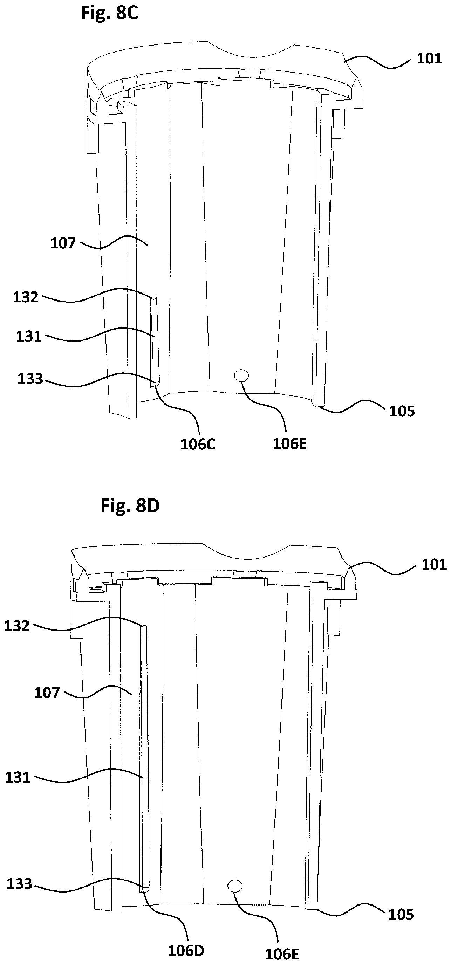

Figs 8A to 8D are schematic illustrations showing alternative embodiments of a protrusion. Figs 8A to 8D show a receptacle 101 with a further end 105. Protrusion 106E is a first embodiment, wherein said protrusion is in the form of a half-sphere. The protrusions 106A - 106D, arranged on protuberance 107, are further embodiments. Fig. 8D shows a protrusion 106D that extends along almost the entire length of the receptacle 101. It can be seen in Figs 8C and 8D that a top surface 131 of protrusions 106C and 106D is slanted between points 132 and 133 on said protrusions. The point 133 is also the point closest to a further imaginary line passing through a centre of the receptacle, i.e., it is the point where the first distance 119 is measured.

### EXAMPLES

The invention is illustrated further by way of examples. The invention is not restricted to the examples.

### Example 1

*Comparative example 1.1*: a structure with a plurality of receptacles is provided. The receptacles have an average length of 20 mm. The receptacles have a cylindrical form. The sidewalls of the receptacles therefore have no protuberances. The sidewalls also have no protrusions. A plurality of containers, in the form of glass syringes, are received in the structure.

*Example 1.2:* a structure similar to the structure of example 1.1 is provided. However, each receptacle, of the structure of example 1.2, has a sidewall with six protrusions arranged equidis-tance around a circumference of the sidewall. The protrusions have the same shape as those shown in Fig. 5. Said protrusions extend a first distance of 0.5 mm into the interior of the receptacle and are positioned at a further distance of 1 mm from the further end (or lower end) of the receptacle. Each protrusion has a surface area of 0.5 mm². A plurality of containers, in the form of glass syringes, are also received in the structure of example 1.2.

*Example 1.3:* a structure similar to the structure of example 1.1 is provided. However, each receptacle, of the structure of example 1.3, has a sidewall with six protuberances arranged equi-distance around a circumference of the sidewall (e.g., Fig. 6). Said protuberances extend into the interior of the receptacle and extend along 97 % of a length of the receptacle. A plurality of containers, in the form of glass syringes, are also received in the structure of example 1.3.

*Example 1.4:* a structure that is a combination of the structure of examples 1.2 and 1.3 is provided. I.e., the receptacles each have six protuberances and six protrusions, wherein the protuberances are arranged on the protuberances (see, e.g., Fig. 5).

Table 1 is a comparison of the structures of examples 1.1 to 1.4.

**Table 1**

| | **1.1** | **1.2** | **1.3** | **1.4** |
|---|---|---|---|---|
| | | | | |

| **Set-up** | | | | |
|---|---|---|---|---|
| Protuberances | No | Yes | No | Yes |
| Protrusions | No | No | Yes | Yes |
| | | | | |

| **Technical effect** | | | | |
|---|---|---|---|---|
| Surface pressure on syringe | +++ | -- | + | --- |
| Contact between syringe and further end of receptacle | +++ | - | + | + |
| Particle count on syringe | +++ | + | - | --- |
| Particle count on receptacle | +++ | + | -- | --- |
| Damage to syringe during insertion/removal | ++ | + | - | - |
| Damage to structure during insertion/removal | + | - | - | - |
| Tilt of syringes | +++ | --- | - | --- |
| Usability for syringes with varying diameter | + | ++ | ++ | +++ |

The technical effects in Table 1 are as follows:
- Surface pressure on syringe: the amount of pressure exerted by the receptacle on the syringe received in said receptacle. A "+" indicates a larger pressure, while a "-" indicates a smaller pressure. It is desired to reduce the pressure.
- Contact between syringe and further end of receptacle: the percentage of the outer surface of a syringe that is in contact with the further end of the receptacle wherein said syringe is received. A "+" indicates a larger percentage, while a "-" indicates a smaller percentage. It is desired to reduce the contact between the outer surface of the syringe and the further end of the sidewall, as the further end has a sharp edge.
- Particle count on syringe: the number of visible and/or sub-visible particles that are present on the exterior and interior surfaces of the syringes. A "+" indicates a larger particle count, and a "-" indicates a smaller particle count. It is desired to reduce the particle count. Visible particles have a size of at least 0.3 mm, and sub-visible particles have a size of more than 10 µm.
- Particle count on receptacle: the number of visible and/or sub-visible particles that are present on the sidewall of a receptacle. A "+" indicates a larger particle count, and a "-" indicates a smaller particle count. It is desired to reduce the particle count. Visible particles have a size of at least 0.3 mm, and sub-visible particles have a size of more than 10 µm.
- Damage to syringe during insertion/removal: the damage to the syringes that occur during insertion and removal of the syringes from the structure. A "+" indicates more damage, and a "-" indicates less damage. It is desired to reduce the damage.
- Damage to structure during insertion/removal: the damage to the structure that occur during insertion and removal of the syringes from the structure. A "+" indicates more damage, and a "-" indicates less damage. It is desired to reduce the damage.
- Tilt of syringes: How much the syringes tilt when received in the receptacles. A "+" indicates a larger tilt, and a "-" indicates less tilt. It is desired to reduce the tilt.
- Usability for syringes with varying diameter: whether the structure can be used for syringes that have a varying diameter (e.g., syringes with caps, wherein the caps have a larger diameter than the diameter of the syringe barrels). A "+" indicates that the structure can be used for syringes with a large variation in diameters, and a "-" indicates that the structure can only be used for syringes with a small variation in diameter. It is desired that the structure can be used for syringes that have a larger diameter variation.

### Example 2

Example is the same as example 1.4, but with the difference that the first distance, that the protrusions extend into the interior of the receptacles, are varied as shown in Table 2.

**Table 2**

| | | | | | |
|---|---|---|---|---|---|
| | **2.1** | **2.2** | **2.3** | **2.4** | **2.5** |
| | | | | | |

| **Set-up** | | | | | |
|---|---|---|---|---|---|
| First distance [mm] | 2 | 1.5 | 1 | 0.5 | 0.1 |
| | | | | | |

| **Technical effect** | | | | | |
|---|---|---|---|---|---|
| Contact between syringe and further end of receptacle | +++ | ++ | + | - | -- |
| Damage to syringe during insertion/removal | ++ | + | - | -- | --- |
| Damage to structure during insertion/removal | ++ | + | - | -- | -- |
| Usability of structure | --- | -- | ++ | +++ | ++ |

The technical effects in Table 2 are the same as described for Table 1.

### Example 3

Example is the same as example 1.4, but with the difference that the further distance between the protrusions and the further ends of the receptacles are varied as shown in Table 3.

**Table 3**

| | | | | | |
|---|---|---|---|---|---|
| | **3.1** | **3.3** | **3.3** | **3.4** | **3.5** |
| | | | | | |

| **Set-up** | | | | | |
|---|---|---|---|---|---|
| Further distance from further end [mm] | 0 | 1 | 2 | 3 | 5 |
| | | | | | |

| **Technical effect** | | | | | |
|---|---|---|---|---|---|
| Contact between syringe and further end of receptacle | --- | --- | -- | - | + |
| Particle count on syringe | --- | --- | -- | -- | + |
| Damage to syringe during insertion/removal | - | - | - | - | + |
| Tilt of syringes | --- | --- | -- | -- | - |

The technical effects in Table 3 are the same as described for Table 1.

### Example 4

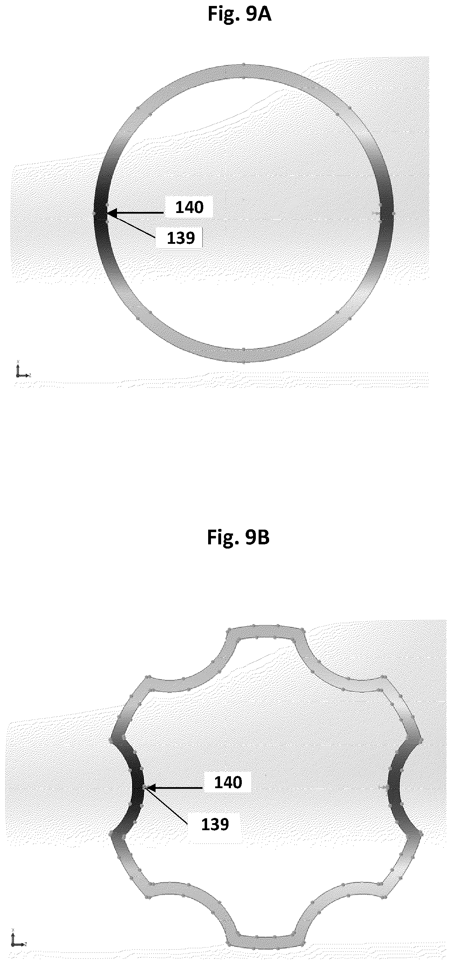

In Example 4 finite element simulations were performed of an elastic deformation of a receptacle when a force is exerted on a sidewall of said receptacle. In example 4.1, a cross-section of the receptacle, made perpendicular to a length of the receptacle, has a circular form (i.e., the receptacle has no protuberances). This is shown in Fig. 9A. In examples 4.2 to 4.4, the receptacle has protuberances. The cross-section of the receptacle, made perpendicular to a length of the receptacle, has a form such as that illustrated in Fig. 9B. In Figs 9A and 9B a force 140 is applied at the position 139 on the sidewall. Table 4 shows the set-up of the examples. The number and dimensions (e.g., width and length) of the protuberances allows for a variation in a stiffness of the receptacle. Stiffness is the extent to which a receptacle (e.g., the sidewall) resists deformation in response to an applied force.

**Table 4**

| | **4.1** | **4.2** | **4.3** | **4.4** |
|---|---|---|---|---|
| | | | | |

| **Set-up** | | | | |
|---|---|---|---|---|
| Form or receptacle cross-section | Circular (see Fig. 9A) | See Fig. 9B | See Fig. 9B | See Fig. 9B |
| Protuberances | No | Yes | Yes | Yes |
| Stiffness [%] | 100 | 91 | 67 | 50 |

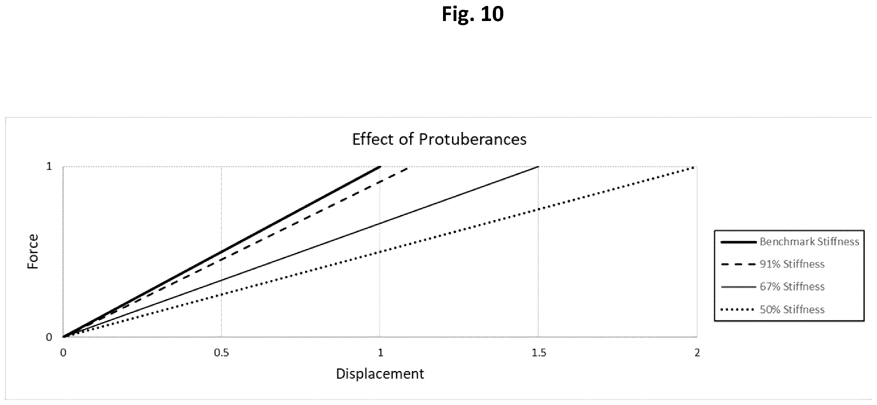

Fig. 10 shows the results of the simulation. It can be seen that for a smaller stiffness a smaller force is required obtain a certain displacement (elastic deformation) of the receptacle sidewall. Stated alternatively, assuming the same force, a larger displacement of the receptacle sidewall is obtained with a decrease in stiffness. Such a reduced stiffness can be obtained by having a sidewall with protuberances. A smaller stiffness is advantageous during insertion and removal of a container from the receptacle, during transport of the containers while received in the structure, as well as to reduce friction between the container and the sidewall of a receptacle.

### TEST METHODS

The test methods which follow were utilized within the context of the invention. Unless stated otherwise, the measurements were conducted at an ambient temperature of 23 °C, an ambient air pressure of 100 kPa (0.986 atm) and a relative air humidity of 50 %.

### Distances, lengths, heights

Distances, lengths, and heights are measured using a calliper. These dimensions are measured as follows. The structure containing the receptacles is placed on a flat surface. The first direction (see, e.g., 134 in Fig. 1) is arranged parallel with the flat surface. The further direction (see, e.g., 115 in Fig. 1) is arranged perpendicular to the flat surface and the first direction.

### REFERENCE LIST

- 100: Structure
- 101: Receptacle
- 102: Sidewall
- 103: Interior of receptacle
- 104: First end of receptacle
- 105: Further end of receptacle
- 106: Protrusion
- 107: Protuberance
- 108: Container
- 109: First end of container
- 110: Further end of container
- 111: Gap
- 112: Protuberance
- 113: Further segment of receptacle
- 114: First surface of structure
- 115: Further direction
- 116: Point of receptacle with larger diameter
- 117: Point of receptacle with smaller diameter
- 118: Further distance
- 119: First distance
- 120: Imaginary rectangle
- 121: Sidewall of neighbouring receptacle
- 122: Gap formed between sidewalls of neighbouring receptacles
- 123: Curves section of sidewall
- 124: First imaginary line
- 125: Second imaginary line
- 126: Centre of receptacle
- 127: Width of protuberance
- 128: Section of sidewall not forming protuberance
- 129: Viewing direction
- 130: Even-further distance
- 131: Top surface of receptacle
- 132: First point on receptacle
- 133: Second point on receptacle
- 134: First direction
- 135: Length of receptacle
- 136: Further imaginary line passing through centre of receptacle
- 137: Point closest to further imaginary line
- 138: Distance between closest point and further imaginary line
- 139: Position where force is applied
- 140: Applied force

## Claims

1. A structure comprising a plurality of receptacles, wherein
a. said receptacles are adapted and arranged for receiving a plurality of containers;
b. each receptacle, of the plurality of receptacles, comprises a sidewall defining an interior of a respective receptacle, wherein the sidewall comprises at least one protrusion extending into the interior of the respective receptacle;
wherein
the at least one protrusion extends a first distance into the interior of the respective receptacle, wherein the first distance is in the range from > 0 to 1.5 mm.

2. The structure according to claim 1, wherein the respective receptacle has a first end and a further end, and wherein the at least one protrusion is arranged closer to the further end than the first end.

3. The structure according to any of the preceding claims, wherein the respective receptacle has a first end and a further end, and the at least one protrusion is arranged at a further distance of *xL* from the further end of the respective receptacle, where *L* is the length of the respective receptacle, and *x* is in the range of 0 to 0.25.

4. The structure according to any of the preceding claims, wherein the at least one protrusion covers a surface area of the sidewall of the respective receptacle that is 15 mm² or less.

5. The structure according to any of the preceding claims, wherein the respective receptacle comprises in the range from 1 to 10 protrusions.

6. The structure according to any of the preceding claims, wherein a diameter of the interior varies along a length of the respective receptacle.

7. The structure according to any of the preceding claims, wherein the at least one protrusion is adapted and arranged to create a gap between a first segment of the sidewall and a first segment of a container when said container is received in the respective receptacle.

8. The structure according to any of the preceding claims, wherein the plurality of receptacles is adapted and arranged to deform when a plurality of containers is received in said receptacles.

9. The structure according to any of the preceding claims, wherein the sidewall of the respective receptacle has at least one section that has a curved surface, and wherein the at least one section forms at least one protuberance that extends into the interior of the respective receptacle.

10. The structure according to claim 9, wherein the at least one protrusion is arranged on the at least one protuberance.

11. The structure according to any of the preceding claims 9 to 10, wherein the at least one protuberance extends along at least 50 % of a length of the respective receptacle.

12. The structure according to any of the preceding claims 9 to 11, wherein the respective receptacle comprises at least two protuberances, and wherein at least 20 % of a circumference of the sidewall of the respective receptacle is in the form of the at least two protuberances.

13. A method for transporting a plurality of containers, comprising the steps of
a. providing a structure according to any of the preceding claims;
b. receiving a plurality of containers in the structure;
c. transporting the structure and the plurality of containers from a first location to a further location.

14. Use of a structure according to any of the preceding claims 1 to 12 for the transport of a plurality of containers received in said structure.

15. Use of a structure according to any of the preceding claims 1 to 12, for at least one or all of the following:
a. reducing contact between the further ends of the plurality of receptacles and the plurality of containers received in said receptacles;
b. reducing contact between neighbouring containers received in the structure;
c. reducing a particle count on the surfaces of the containers received in the structure;
d. reducing damage to the containers when received in the structure.
